(19)

Europäisches
Patentamt
European
Patent Office
Office européen
des brevets

(11) **EP 2 692 372 B1**

(12) **EUROPEAN PATENT SPECIFICATION**

(45) Date of publication and mention
of the grant of the patent:
**06.07.2016 Bulletin 2016/27**

(21) Application number: **12763469.9**

(22) Date of filing: **29.03.2012**

(51) Int Cl.:
*A61M 1/36* *(2006.01)*    *B01J 20/26* *(2006.01)*
*B01J 20/28* *(2006.01)*    *B01J 20/32* *(2006.01)*

(86) International application number:
**PCT/JP2012/058294**

(87) International publication number:
**WO 2012/133609 (04.10.2012 Gazette 2012/40)**

(54) **BLOOD-PURIFYING COLUMN**

BLUTREINIGUNGSSÄULE

COLONNE DE PURIFICATION DE SANG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.03.2011   JP 2011076156**

(43) Date of publication of application:
**05.02.2014   Bulletin 2014/06**

(73) Proprietor: **Toray Industries, Inc.
Tokyo 103-8666 (JP)**

(72) Inventor: **OKUDA, Hiroyuki
Aichi444-8522 (JP)**

(74) Representative: **Kador & Partner
Corneliusstraße 15
80469 München (DE)**

(56) References cited:
**EP-A1- 1 312 387      EP-A1- 2 067 495
WO-A1-00/38763      GB-A- 2 213 056
JP-A- 9 239 022      JP-A- 62 243 561
JP-A- 2003 225 304      JP-A- 2008 237 893
JP-B- 1 016 389      JP-B2- 2 853 452
JP-Y2- 60 013 481**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a blood purification column.

BACKGROUND ART

Since the beginning of use of hemodialysis for treatment of acute renal failure, blood purification therapy, wherein extracorporeal circulation of blood is carried out to directly remove unwanted substances and causative substances of diseases, has been drawing attention. For example, in treatment of sepsis and leukemia, a therapy wherein a blood purification column filled with a polymyxin-immobilized fiber (Patent Documents 1 and 2) as an adsorbent carrier is used to perform extracorporeal circulation of blood to remove endotoxin from blood of the patient has been practically used. In addition, in recent years, attempts are being made to remove leukocytes, granulocytes, cytokines, LDL cholesterol and the like in blood purification therapy to cure blood diseases, ulcerative colitis, rheumatic diseases, hypercholesterolemia and the like.

The amount of blood to be subjected to extracorporeal circulation can be controlled by the size of the blood purification column, that is, the blood capacity. On the other hand, for securing safety of the patient, the amount of blood that can be removed from the body at once is limited, and the blood capacity is therefore an important parameter that needs to be appropriately selected depending on the body weight of the patient and the like. Therefore, products with various column sizes (Toraymyxin (registered trademark); Toray Industries, Inc.), such as those having blood capacities of 40 mL and 135 mL, are commercially available as columns for adsorption of endotoxin, so that adult patients can select the blood capacity depending on the body weight and pathological conditions.

In a blood purification column having a pipe, that is, a blood flow tube, in the central portion of the column, wherein blood flows in the direction vertical to the longitudinal direction of the column, it is said that downsizing of the column for the purpose of decreasing the blood capacity also requires reduction in the diameter of the blood flow tube. This is because, since reduction in the blood capacity also causes a decrease in the flow rate of blood during extracorporeal circulation, the blood flow tube needs to be thinner in order to keep the linear velocity of blood that flows from the blood flow tube within a certain range.

For filling an adsorbent carrier into a blood purification column having a pipe, that is, a blood flow tube in the central portion of the column, wherein blood flows in the direction vertical to the longitudinal direction of the column, a method wherein an appropriate amount of an adsorbent carrier molded into a sheet-like shape is wound around the blood flow tube and the resultant is then inserted into the column is commonly used. However, it is said that the operation of winding a sheet-like adsorbent carrier (for example, knitted fabric) around the blood flow tube needs to be fully manually carried out since a wet adsorbent carrier promotes corrosion of the machine and mechanical operation in a clean room is often avoided in the field of medicine.

PRIOR ART DOCUMENTS

[Patent Documents]

[Patent Document 1] JP 1671925 B
[Patent Document 2] JP 2853452 B
EP1066843A1

SUMMARY OF THE INVENTION

PROBLEMS TO BE SOLVED BY THE INVENTION

However, no report has been made at all on a downsized blood purification column having a blood capacity of 10 mL or less that can be suitably used for treatment of children and the like who have only 200 to 800 mL of blood in the body. On the other hand, development of a blood purification column that can be used for children has been strongly demanded by pediatricians.

The reason why a blood purification column having a blood capacity of 10 mL or less has not been developed is that, in order to reduce the blood capacity of the blood purification column to 10 mL or less, it is thought, according to calculation, that the diameter of the blood flow tube needs to be extremely small. This increases the risk of generation of a thrombus and the like in the blood flow tube to cause clogging of the blood flow tube, and does not allow manual operation of winding an adsorbent carrier around the blood flow tube, which are problematic.

In view of this, the present invention aims to provide a downsized blood purification column having a blood capacity of 10 mL or less, which has a low risk of clogging of the blood flow tube and allows manual winding of an adsorbent carrier.

## MEANS FOR SOLVING THE PROBLEMS

That is, the present invention provides the blood purification column according to (1) to (7) below.

(1) A blood purification column comprising:

a cylindrical body;
a first header that closes one end of the cylindrical body and has a first blood channel that communicates with the inside of the cylindrical body;
a second header that closes the other end of the cylindrical body and has a second blood channel that communicates with the inside of the cylindrical body;
an adsorbent carrier contained in the cylindrical body;
a first end plate provided at the first-header-side end of the cylindrical body;
a second end plate provided at the second-header-side end of the cylindrical body; and
a cylindrical blood flow tube that extends in the central portion of the cylindrical body from the first end plate to the second end plate, a plurality of openings being formed in the circumferential surface of the cylindrical blood flow tube;

wherein:

one end of the blood flow tube communicates with the first blood channel;
the other end of the blood flow tube is closed;
the first end plate is installed such that its outer circumferential surface closely contacts with the inner circumferential surface of the cylindrical body;
a gap is provided between the outer circumferential surface of the second end plate and the inner circumferential surface of the cylindrical body;
the ratio of the outer diameter of the blood flow tube in the cross-section vertical to the longitudinal direction, D1, to the inner diameter of the cylindrical body in the cross-section vertical to the longitudinal direction, D2, is 0.35 to 0.50; and
the blood capacity is 6 to 10 mL.

(2) The blood purification column according to (1), wherein the residence time is 0.9 to 10.0 minutes when the flow rate of inflowing blood is 3 to 10 mL/min.
(3) The blood purification column according to (1) or (2), wherein the open area ratio of the blood flow tube is 15 to 85%.
(4) The blood purification column according to (1) to (3), wherein the adsorbent carrier is a knitted fabric.
(5) The blood purification column according to (4), wherein the knitted fabric is wound around the blood flow tube.
(6) The blood purification column according to any one of (1) to (5), wherein the adsorbent carrier is composed of an antibiotic-immobilized fiber.
(7) The blood purification column according to (6), wherein the antibiotic is polymyxin.

The blood purification column is especially useful for children since, in spite of its reduced blood capacity of 10 mL or less, a thrombus is less likely to be generated in the blood flow tube, and the operation of winding an adsorbent carrier around the blood flow tube can be easily carried out.

## EFFECT OF THE INVENTION

The present invention can provide a safe blood purification column having a reduced blood capacity of 10 mL or less wherein the risk of occurrence of clogging in the blood flow tube and other portions is largely suppressed, which column can be suitably used for treatment of children. Further, the present invention conveniently allows manual operation of winding an adsorbent carrier in the production process of the downsized blood purification column.

BRIEF DESCRIPTION OF THE DRAWINGS

Fig. 1 is a schematic diagram illustrating a cross-section of the blood purification column of the first embodiment of the present invention, which cross-section is parallel to the longitudinal direction.
Fig. 2 is a schematic diagram illustrating a state where an adsorbent carrier which is a knitted fabric is wound around the blood flow tube constituting the blood purification column of the first embodiment of the present invention.

BEST MODE FOR CARRYING OUT THE INVENTION

Preferred embodiments of the present invention are described below in detail by reference to the drawings, but the present invention is not limited to these modes. The ratios in the drawings are not necessarily the same as those in the description.

The blood purification column of the present invention is a blood purification column comprising:

a cylindrical body;
a first header that closes one end of the cylindrical body and has a first blood channel that communicates with the inside of the cylindrical body;
a second header that closes the other end of the cylindrical body and has a second blood channel that communicates with the inside of the cylindrical body;
an adsorbent carrier contained in the cylindrical body;
a first end plate provided at the first-header-side end of the cylindrical body;
a second end plate provided at the second-header-side end of the cylindrical body;and
a cylindrical blood flow tube that extends in the central portion of the cylindrical body from the first end plate to the second end plate, a plurality of openings being formed in the circumferential surface of the cylindrical blood flow tube;

wherein:

one end of the blood flow tube communicates with the first blood channel;
the other end of the blood flow tube is closed;
the first end plate is installed such that its outer circumferential surface closely contacts with the inner circumferential surface of the cylindrical body;
a gap is provided between the outer circumferential surface of the second end plate and the inner circumferential surface of the cylindrical body;
the ratio of the outer diameter of the blood flow tube in the cross-section vertical to the longitudinal direction, D1, to the inner diameter of the cylindrical body in the cross-section vertical to the longitudinal direction, D2, is 0.35 to 0.50; and
the blood capacity is 6 to 10 mL.

Since the blood purification column of the present invention has a blood capacity of 6 to 10 mL, it can be suitably used in treatment of a child. The "child" herein means a human individual from birth to the age of about 6 years old having a body weight within the range of 3 to 10 kg, wherein the amount of blood in the body is within the range of 200 to 800 mL. The "child" include the so-called neonate, suckling and infant.

Blood vessels in children are generally thin, and show large variation among individuals. In some cases, treatment is carried out for children having blood vessels which are much thinner than those in adults. Therefore, in treatment of a child using a blood purification column, the flow rate of blood needs to be suppressed in sufficient consideration of the physical load on the child. On the other hand, too much suppression of the flow rate of blood causes problems such as (i) stopping of blood in the column and loss of the pharmacological effect of an anticoagulant, leading to coagulation of the blood and clogging of the blood purification column; and (ii) elongation of the duration of the procedure, which increases the physical load on the child. When the column is used, the flow rate of blood removed from the body is preferably 1 mL/min. per kg body weight of the child. That is, the flow rate of blood removed from the body of a child having a body weight within the range of 3 to 10 kg is preferably 3 to 10 mL/min.

After removal from the body, blood that flowed into the blood purification column passes through the blood flow tube and then flows into the adsorbent carrier. In cases where the flow rate of the blood is too high, problems such as (i) activation of the blood due to the shear stress caused in the column, which causes clogging of the adsorbent carrier; (ii) insufficient contact time between the blood and the adsorbent carrier, which prevents production of the expected performance; and/or the like occurs.

Because of these reasons, in cases where the flow rate of blood removed from the body, that is, the flow rate of blood that flows into the blood purification column, is 3 to 10 mL/min., the time required for the blood to pass through the adsorbent carrier, that is, the residence time, is preferably 0.9 to 10.0 minutes.

The "residence time" herein means the time calculated according to the Equation 1 below.

$$\text{Residence time } t = (D2-D1)\times AL/(v\times 100) \ldots \text{ Equation 1}$$

AL: longitudinal length (mm) of the portion covered with the adsorbent carrier in the blood flow tube

D 1: outer diameter (mm) of the blood flow tube

D2: inner diameter (mm) of the cylindrical body containing the adsorbent carrier therein in the cross-section vertical to the longitudinal direction

v: flow rate of blood that flows into the blood purification column = 10 mL/min.

The "adsorbent carrier" is preferably an aggregate of fibers. Examples of the aggregate of fibers herein include knitted fabrics, woven fabrics and nonwoven fabrics, and, in view of simplicity of filling by manual operation, knitted fabrics are preferred.

Examples of the material of the fibers include polyolefins such as polyethylene and polypropylene; polyesters such as polyethylene terephthalate and polybutylene terephthalate; polysulfone polymers such as poly(p-phenylene ether sulfone); polyetherimide; polyimide; polyamide; polyether, polyphenylene sulfide; polystyrene; and polyacrylonitrile polymers. In view of modifiability of the surface of a water-insoluble carrier by amidomethylation and the like, polystyrene is preferred.

Examples of the structure of the fiber include single yarns composed of a single type of polymer; and composite fibers such as those of the core/sheath type, sea/island type and side-by-side type. In particular, multicore sea/island type composite fibers wherein the core is polypropylene, the sheath is polystyrene, and the sea is polyethylene terephthalate; and sea/island type composite fibers wherein the island is polypropylene and the sea is polystyrene; are preferred. Further, it is also preferred to give strength and heat resistance to the fiber by introducing a crosslinked structure with formaldehyde or paraformaldehyde or by coating the surface with another polymer.

The incidence rate of Gram-negative infection in children is equivalent to that in adults, and a novel therapeutic method for children needs to be provided, so that the above-described fiber is preferably an antibiotic-immobilized fiber, more preferably a polymyxin-immobilized fiber, wherein the antibiotic is polymyxin.

Examples of the polymyxin include polymyxin A, polymyxin B (polymyxin B1 or polymyxin B2), polymyxin D1 and polymyxin E1, and the polymyxin is preferably polymyxin B.

Examples of the specific method for immobilizing polymyxin on the fiber include a method wherein polymyxin is reacted with a polystyrene having, as a reactive functional group, a chloroacetamidomethyl group, that is, chloroacetamidomethylated polystyrene.

Examples of the reactive functional group, in addition to a haloacetamidomethyl group such as chloroacetamidomethyl, include an active halogen group such as halomethyl, haloacetyl or halogenated alkyl; epoxide group; carboxyl group; isocyanate group; thioisocyanate group; and acid anhydride group.

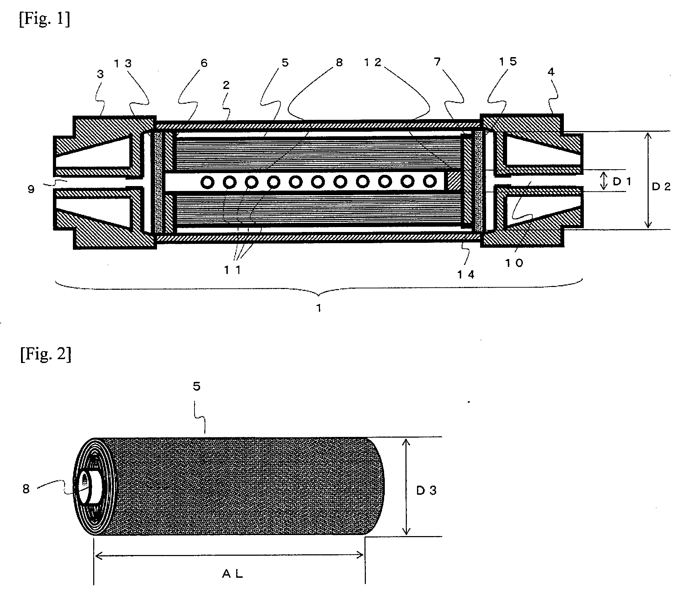

The blood purification column 1 illustrated in Fig. 1 is composed of:

a cylindrical body 2;
a first header 3 that closes one end of the cylindrical body 2;
a second header 4 that closes the other end of the cylindrical body 2;
an adsorbent carrier 5 contained in the cylindrical body 2;
a first end plate 6 provided at the first-header-3-side end of the cylindrical body 2;
a second end plate 7 provided at the second-header-4-side end of the cylindrical body 2; and
a blood flow tube 8 that extends from the first end plate 6 to the second end plate 7.

The first header 3 and the second header 4 have a first blood channel 9 and a second blood channel 10, respectively, that communicate with the inside of the cylindrical body 2. The first end plate 6 and the second end plate 7 are provided at both end surfaces of the adsorbent carrier 5 in the longitudinal direction. The blood flow tube 8 is provided at the central portion of the cylindrical body 2.

A plurality of openings 11, through which blood flows, are formed in the circumferential surface of the cylindrical blood flow tube 8, and one end of the blood flow tube 8 opens to the outside of the first end plate 6 and communicates with the first blood channel 9. The other end of the blood flow tube 8 is closed by a closing section 12.

The first end plate 6 is installed such that its outer circumferential surface closely contacts with the inner circumferential surface of the cylindrical body 2. A filter 13 is installed on the outside surface of the first end plate 6. When blood flows into the cylindrical body 2 from the first blood channel 9, the blood first passes through the filter 13 and then flows into the blood flow tube 8.

The second end plate 7 is installed in the cylindrical body 2 such that a gap 14 is formed between the outer circumferential surface of the second end plate 7 and the inner circumferential surface of the cylindrical body 2, which gap allows the blood to flow therethrough. The gap 14 communicates with the second blood channel 10. On the outer surface of the second end plate 7, a filter 15 is installed. When blood flows into the cylindrical body 2 from the first blood channel 9, the blood that flowed into the blood flow tube 8 passes through the plurality of openings 11 on the blood flow tube 8, and flows into the gap portion of the adsorbent carrier 5, followed by flowing in the adsorbent carrier 5 in the direction to its circumference. During this, the substances to be removed are removed by adsorption by the adsorption removal capacity of the adsorbent carrier 5. Blood that has passed through the adsorbent carrier 5 flows through the gap between the outer circumferential surface of the adsorbent carrier 5 and the inner circumferential surface of the cylindrical body 2, and flows out from the gap 14, followed by passing through the filter 15 and reaching the second blood channel 10.

The blood purification column 1 illustrated in Fig. 1 is a column having the blood flow tube 8, wherein blood flows in the cylindrical body 2 in the direction vertical to the longitudinal direction of the blood purification column 1. Alternatively, the blood may flow into the cylindrical body 2 from the second blood channel 10, instead of flowing into the cylindrical body 2 from the first blood channel 9 as described above.

The adsorbent carrier 5 constituting the blood purification column 1 is a knitted fabric wound around the blood flow tube 8 as illustrated in Fig. 2.

The inner diameter of the cylindrical body containing the adsorbent carrier therein, $D_2$, is preferably 15 to 25 mm, more preferably 15 to 20 mm, for setting the blood capacity within an appropriate range.

The outer diameter of the blood flow tube, $D_1$, is preferably 6 to 10 mm, more preferably 7 to 9 mm, for setting the blood capacity within an appropriate range while suppressing clogging of the blood flow tube.

From the above reasons, the ratio of the outer diameter of the blood flow tube in the cross-section vertical to the longitudinal direction, $D_1$, to the inner diameter of the cylindrical body in the cross-section vertical to the longitudinal direction, $D_2$, that is, the value $D_1/D_2$, is preferably 0.40 to 0.47.

In the blood purification column of the present invention, the blood flow tube is required to have a "cylindrical" shape. The term "cylindrical" herein means a hollow cylinder, that is, a round tube or a similar shape in which the shape of the cross-section vertical to the longitudinal direction is elliptical or polygonal. The shape of the cross-section vertical to the longitudinal direction of the blood flow tube is preferably a true circle, but, in cases where the shape is elliptical or polygonal, the diameter of the true circle having the same area as the cross-section can be regarded as the $D_1$ described above. The thickness of the blood flow tube does not need to be uniform, and, for example, both ends may be thinner than the middle portion, or the middle portion may be narrow.

In terms of the wall thickness of the cylindrical blood flow tube, in cases where the thickness is small, the strength of the blood flow tube cannot be secured, while in cases where the thickness is too large, clogging of the openings is likely to occur, so that the thickness is preferably 1 to 2 mm. That is, in cases where the shape of the blood flow tube is cylindrical, the inner diameter is preferably 2 to 4 mm smaller than the $D_1$ described above, more preferably 3 mm smaller than the $D_1$ described above.

The shape of the plurality of openings formed in the circumferential surface of the blood flow tube is preferably circular, and a taper is more preferably provided around the circle.

The "cylindrical body" constituting the blood purification column of the present invention means a hollow cylinder, and the "cylindrical body" also includes a shape of a cylinder wherein the shape of the hollow portion in the cross-section vertical to the longitudinal direction is elliptical or polygonal. The shape of the hollow portion in the cross-section vertical to the longitudinal direction is preferably an ellipse or polygon that is close to a true circle, more preferably a true circle. In cases where the shape is elliptical or polygonal, the diameter of the true circle having the same area as the cross-section can be regarded as the $D_2$ described above. The thickness of the cylindrical body does not need to be uniform, and, for example, both ends may be thinner than the middle portion, or the middle portion may be narrow. As long as the shape of the hollow portion in the cross-section vertical to the longitudinal direction is close to a true circle, the outer shape may even be a rectangular parallelepiped or the like.

Examples of the materials of the cylindrical body, blood flow tube, header and the like constituting the blood purification column of the present invention include polycarbonate, polyvinyl chloride, polyacrylonitrile, polyester, polyurethane, polystyrene, polyethylene, polypropylene and polyvinylidene fluoride. Polypropylene is preferred.

The blood purification column of the present invention is required to have a blood capacity of 6 to 10 mL. The "blood capacity" herein means the volume calculated according to the Equation 2 below.

$$\text{Blood capacity (mL)} = (W1-W2) \div a \ldots \text{Equation 2}$$

W1: total weight (g) of the blood purification column filled with physiological saline
W2: total weight (g) of the blood purification column after removal of physiological saline contained therein
a: specific gravity (g/mL) of the physiological saline used

For determination of W2, air was sent into the blood purification column using a roller pump at a flow rate of 10 mL/min. for 5 minutes to discharge physiological saline, and the blood purification column was then tapped, followed by sending air at a flow rate of 10 mL/min. for 5 minutes and measuring the total weight of the blood purification column.

The open area ratio of the blood flow tube is preferably 15 to 85%, more preferably 35 to 65%, in order to suppress a pressure increase in the blood during extracorporeal circulation.

The "open area ratio" means the value calculated according to the Equation 3 below.

$$\text{Open area ratio } OR(\%) = (TOA/SA) \times 100 \ldots \text{Equation 3}$$

TOA: total open area ($mm^2$) of the plurality of openings
SA: area ($mm^2$) of the portion covered with the adsorbent carrier on the outer surface of the blood flow tube

The SA herein means the volume calculated according to the Equation 4 below.

$$SA\ (mm^2) = AL \times D1 \times \pi \ldots \text{Equation 4}$$

AL: length (mm) of the portion covered with the adsorbent carrier in the longitudinal direction of the blood flow tube
D 1: outer diameter (mm) of the blood flow tube

EXAMPLES

The present invention is described below in detail by way of Examples, but the present invention is not limited thereto.

(Preparation of Adsorbent Carrier)

Multicore sea/island type composite fibers (number of islands, 16; single yarn fineness, 2.6 deniers; tensile strength, 2.9 g/d; elongation percentage, 50%; filament number, 42) were prepared by melt spinning using, as the island component, 50 parts by weight of insoluble polypropylene (Prime Polypro (registered trademark) J105WT, Prime Polymer Co., Ltd.), and, as the sea component, a mixture of 45 parts by weight of polystyrene (PSJ-Polystyrene 679, PS Japan Corporation) and 5 parts by weight of polypropylene (Prime Polypro J105WT, Prime Polymer Co., Ltd.). After doubling with two fibers, a tubular knit was prepared (hereinafter referred to as polypropylene-reinforced polystyrene fiber).

In a reaction vessel, 120 g of concentrated sulfuric acid and 120 g of nitrobenzene were mixed, and 0.3 g of paraformaldehyde was added to the resulting mixture and melt at room temperature. While the resulting mixture was cooled in an ice bath, 18 g of N-methylol-2-chloroacetamide was added thereto dividedly in 3 times for 10 minutes, and the resulting mixture was stirred at room temperature for 45 minutes, to prepare a haloacetamidomethylation agent.

In the haloacetamidomethylation agent, 10 g of the polypropylene-reinforced polystyrene fiber was immersed, and the resultant was stirred for 2 hours, to obtain a haloacetamidomethylated fiber.

The whole haloacetamidomethylated fiber obtained was washed with 180 mL of nitrobenzene and 180 mL of distilled water. Thereafter, 10 g of 6 N sodium hydroxide solution was added thereto for neutralization. Subsequently, the haloacetamidomethylated fiber was further washed 10 times with 200 mL of methanol and then once with 2000 mL of warm water.

The haloacetamidomethylated fiber after washing, 200 mg of polymyxin B sulfate and 130 mL of distilled water were placed in a reaction vessel, and the resultant was stirred at room temperature for 30 minutes, followed by adding 9 g of 0.1 N aqueous sodium hydroxide solution thereto and stirring the resultant for an additional hour. After completion of the stirring, the mixture in the reaction vessel was neutralized with 1 N hydrochloric acid, and washing was performed 3 times with 130 mL of distilled water, to obtain a polymyxin B-immobilized fiber.

From the obtained polymyxin B-immobilized fiber, its knitted fabric was prepared such that its longitudinal width was 47 mm.

A polypropylene blood flow tube 8 having an outer diameter (D1) of 8 mm, inner diameter of 5 mm and length of 47 mm was prepared. On the circumferential surface of the blood flow tube 8, a plurality of circular openings were formed at regular intervals. The area of each opening was 23.2 $mm^2$, and, since 20 openings were formed, TOA was 464 $mm^2$.

The prepared knitted fabric was manually wound around the outer circumferential surface of the blood flow tube 8, to prepare an adsorbent carrier 5. The winding was performed such that the weight of the adsorbent carrier 5 was 3 g (hereinafter referred to as adsorbent carrier 5-1). The outer diameter D3 of the adsorbent carrier 5-1 after the winding, which was hollow cylinder-shaped, was 15 mm.

In addition to adsorbent carrier 5-1, adsorbent carrier 5-2 and adsorbent carrier 5-3 were prepared such that the weight of the adsorbent carrier was 4 g and 5 g, respectively. Since AL was 47 mm in all of adsorbent carriers 5-1 to 5-3, SA was 1181 $mm^2$, and, as a result, the open area ratio OR was 39% in all cases.

(Preparation of Blood Purification Column 1)

Two polypropylene cylindrical bodies 2 having an outer diameter of 25 mm, inner diameter (D2) of 20 mm and length of 50 mm were prepared. To both ends of the prepared adsorbent carrier 5-1, a preliminarily prepared polypropylene first end plate 6 and second end plate 7 were attached, and the resultant was inserted into the cylindrical body 2 and stored therein, followed by attaching each of a polypropylene filter 13 and filter 15. Thereafter, a preliminarily prepared polypropylene header 3 and header 4 were attached to both ends of the cylindrical body 2, and absence of leakage was confirmed, to complete a blood purification column (hereinafter referred to as blood purification column 1). A total of three blood purification columns 1 were prepared, and designated blood purification columns 1-1, 1-2 and 1-3, respectively.

(Preparation of Blood Purification Column 2)

The same operations as described above were carried out except that adsorbent carrier 5-2 was used instead of adsorbent carrier 5-1, to complete blood purification columns (hereinafter referred to as blood purification columns 2). A total of three blood purification columns 2 were prepared, and designated blood purification columns 2-1, 2-2 and 2-3, respectively.

(Preparation of Blood Purification Column 3)

The same operations as described above were carried out except that adsorbent carrier 5-3 was used instead of adsorbent carrier 5-1, to complete blood purification columns (hereinafter referred to as blood purification columns 3). A total of three blood purification columns 3 were prepared, and designated blood purification columns 3-1, 3-2 and 3-3, respectively.

Each of the cylindrical body 2 and the blood flow tube 8 used for preparation of blood purification columns 1 to 3 had the same size. Therefore, the ratio of the outer diameter of the blood flow tube 8 in the cross-section vertical to the longitudinal direction, D1, to the inner diameter of the cylindrical body in the cross-section vertical to the longitudinal direction, D2, that is, the value D1/D2, was 0.40 in all cases.

(Measurement of Blood Capacity)

The blood capacity of each of the prepared blood purification columns 1 to 3 was measured. The results are shown in Table 1. Since the weight of 1 mL of the physiological saline used was 1.016 g, this value was used as a (g/mL).

[Table 1]

| Blood purification column number | Weight of adsorbent carrier [g] | W1 [g] | W2 [g] | W1-W2 [g] | Blood capacity [mL] | Average blood capacity [mL] |
|---|---|---|---|---|---|---|
| 1-1 | 3 | 89.18 | 79.11 | 10.07 | 9.91 | |
| 1-2 | 3 | 89.11 | 78.83 | 10.28 | 10.1 | 10.0 |
| 1-3 | 3 | 88.95 | 78.78 | 10.17 | 10.0 | |
| 2-1 | 4 | 89.10 | 81.59 | 7.51 | 7.39 | |

(continued)

| Blood purification column number | Weight of adsorbent carrier [g] | W1 [g] | W2 [g] | W1-W2 [g] | Blood capacity [mL] | Average blood capacity [mL] |
|---|---|---|---|---|---|---|
| 2-2 | 4 | 89.27 | 82.36 | 6.91 | 6.80 | 7.2 |
| 2-3 | 4 | 89.02 | 81.44 | 7.58 | 7.46 | |
| 3-1 | 5 | 89.01 | 83.33 | 5.68 | 5.59 | 5.7 |
| 3-2 | 5 | 89.28 | 83.22 | 6.06 | 5.96 | |
| 3-3 | 5 | 88.93 | 83.26 | 5.67 | 5.58 | |

(Evaluation of Endotoxin Adsorption Capacity)

By the same operations as described above, a total of three blood purification columns 2 were prepared, and the columns were designated blood purification column 2-4, 2-5 and 2-6. Each of these blood purification columns 2 was filled with physiological saline, and sterilized at 117°C for 90 minutes.

Endotoxin-containing serum was prepared in an amount of 150 mL such that the endotoxin concentration was 10 ng/mL.

A sterilized blood purification column 2-4 was connected to the blood line, and 500 mL of physiological saline was sent into the blood purification column 2-4 at a flow rate of 100 mL/min. to wash the inside of the blood purification column 2-4 and the adsorbent carrier 5-2. Thereafter, 60 mL of the endotoxin-containing serum was sent into the blood purification column 2-4 filled with physiological saline, and the liquid inside the column was discharged. The remaining 90 mL of endotoxin-containing serum was perfused at a flow rate of 10 mL/min. for 4 hours. The temperature of the endotoxin-containing serum during the perfusion was kept at 37°C.

The endotoxin-containing serum after perfusion was collected and 10-fold diluted with distilled water for injection. The resulting dilution was then subjected to heat treatment at 70°C for 10 minutes, and the gel time was determined using a Toxinometer. The endotoxin concentration (ng/mL, hereinafter referred to as sample concentration) after perfusion was determined based on a preliminarily prepared calibration curve.

From the determined sample concentration, the endotoxin removal rate was calculated according to the Equation 4 below.

$$\{(\text{initial concentration - sample concentration}) / \text{initial concentration}\} \times 100$$

$$(\%) \ldots \text{Equation 4}$$

The initial concentration was 10 ng/mL as described above. The results are shown in Table 2.

A sterilized blood purification column 2-5 and blood purification column 2-6 were evaluated similarly to the blood purification column 2-4 to calculate the endotoxin removal rate for each column. The results are shown in Table 2. No increase in the pressure due to clogging occurred at all during the perfusion for 4 hours in any of the blood purification columns 2-4 to 2-6.

[Table 2]

| Blood purification column number | Endotoxin removal rate [%] |
|---|---|
| 2-4 | 82 |
| 2-5 | 80 |
| 2-6 | 80 |

It is said that the endotoxin removal rate in blood purification therapy is preferably not less than 60%. On the other hand, all of the blood purification columns 2-4 to 2-6 showed endotoxin removal rates of as high as not less than 80%. From these results, it is clear that the blood purification column of the present invention having a reduced blood capacity of not more than 10 mL is a safe blood purification column specialized in treatment of children, wherein there is no risk of clogging of the blood flow tube.

INDUSTRIAL APPLICABILITY

The present invention can be used as a blood purification column in the field of medicine.

DESCRIPTION OF SYMBOLS

1, Blood purification column; 2, Cylindrical body; 3, First header; 4, Second header; 5, Adsorbent carrier; 6, First end plate; 7, Second end plate; 8, Blood flow tube; 9, First blood channel; 10, Second blood channel; 11, Openings; 12, Closing section; 13, Filter; 14, Gap; 15, Filter

**Claims**

1.   A blood purification column (1) comprising:

   a cylindrical body (2);
   a first header (3) that closes one end of said cylindrical body (2) and has a first blood channel (9) that communicates with the inside of said cylindrical body (2);
   a second header (4) that closes the other end of said cylindrical body (2) and has a second blood channel (10) that communicates with the inside of said cylindrical body (2);
   an adsorbent carrier (5) contained in said cylindrical body (2);
   a first end plate (6) provided at the first-header-side end of said cylindrical body (2);
   a second end plate (7) provided at the second-header-side end of said cylindrical body (2); and
   a cylindrical blood flow tube (8) that extends in the central portion of said cylindrical body (2) from said first end plate (6) to said second end plate (7), a plurality of openings (11) being formed in the circumferential surface of said cylindrical blood flow tube (8);

   wherein:

   one end of said blood flow tube (8) communicates with said first blood channel(9);
   the other end of said blood flow tube (8) is closed;
   said first end plate (6) is installed such that its outer circumferential surface closely contacts with the inner circumferential surface of said cylindrical body (2);
   a gap (14) is provided between the outer circumferential surface of said second end plate (7) and the inner circumferential surface of said cylindrical body (2);
   the ratio of the outer diameter of said blood flow tube (8) in the cross-section vertical to the longitudinal direction, D1, to the inner diameter of said cylindrical body (2) in the cross-section vertical to the longitudinal direction, D2, is 0.35 to 0.50; and
   the blood capacity is 6 to 10 ml.

2.   The blood purification column according to claim 1, wherein the residence time is 0.9 to 10.0 minutes when the flow rate of inflowing blood is 3 to 10 ml/min.

3.   The blood purification column according to claim 1 or 2, wherein the open area ratio of said blood flow tube (8) is 15 to 85%.

4.   The blood purification column according to claims 1 to 3, wherein said adsorbent carrier (5) is a knitted fabric.

5.   The blood purification column according to claim 4, wherein said knitted fabric is wound around said blood flow tube (8).

6.   The blood purification column according to any one of claims 1 to 5, wherein said adsorbent carrier (5) is composed of an antibiotic-immobilized fiber.

7.   The blood purification column according to claim 6, wherein said antibiotic is polymyxin.

**Patentansprüche**

1. Blutaufreinigungssäule (1), umfassend:

   einen zylindrischen Körper (2);
   ein erstes Kopfstück (3), das ein Ende des zylindrischen Körpers (2) verschließt und einen ersten Blutkanal (9) aufweist, der mit dem Inneren des zylindrischen Körpers (2) kommuniziert;
   ein zweites Kopfstück (4), das das andere Ende des zylindrischen Körpers (2) verschließt und einen zweiten Blutkanal (10) aufweist, der mit dem Inneren des zylindrischen Körpers (2) kommuniziert;
   einen Adsorptionsmittelträger (5), der in dem zylindrischen Körper (2) enthalten ist;
   eine erste Endplatte (6), die an dem Ende auf der Seite des ersten Kopfstücks von dem zylindrischen Körper (2) bereitgestellt ist;
   eine zweite Endplatte (7), die an dem Ende auf der Seite des zweiten Kopfstücks von dem zylindrischen Körper (2) bereitgestellt ist; und
   ein zylindrisches Blutströmungsrohr (8), das sich in dem zentralen Teil des zylindrischen Körpers (2) von der ersten Endplatte (6) zur zweiten Endplatte (7) erstreckt, wobei eine Vielzahl von Öffnungen (11) in der umlaufenden Oberfläche des zylindrischen Blutströmungsrohrs (8) ausgebildet ist;

   wobei:

   ein Ende des Blutströmungsrohrs (8) mit dem ersten Blutkanal (9) kommuniziert;
   das andere Ende des Blutströmungsrohrs (8) verschlossen ist;
   die erste Endplatte (6) so angebracht ist, dass ihre äußere umlaufende Oberfläche mit der inneren umlaufenden Oberfläche des zylindrischen Körpers (2) in einem engen Kontakt steht;
   ein Abstand (14) zwischen der äußeren umlaufenden Oberfläche der zweiten Endplatte (7) und der inneren umlaufenden Oberfläche des zylindrischen Körpers (2) bereitgestellt ist;
   das Verhältnis von dem Außendurchmesser des Blutströmungsrohrs (8) im Querschnitt vertikal zur Längsrichtung, D1, zu dem Innendurchmesser des zylindrischen Körpers (2) im Querschnitt vertikal zur Längsrichtung, D2, 0,35 bis 0,50 beträgt; und
   die Blutkapazität 6 bis 10 ml beträgt.

2. Blutaufreinigungssäule nach Anspruch 1, wobei die Verweildauer 0,9 bis 10,0 Minuten beträgt, wenn die Strömungsgeschwindigkeit von einströmendem Blut 3 bis 10 ml/min ist.

3. Blutaufreinigungssäule nach Anspruch 1 oder 2, wobei das Verhältnis der offenen Flächen des Blutströmungsrohrs (8) 15 bis 0,85 % beträgt.

4. Blutaufreinigungssäule nach einem der Ansprüche 1 bis 3, wobei der Adsorptionsmittelträger (5) eine Maschenware ist.

5. Blutaufreinigungssäule nach Anspruch 4, wobei die Maschenware um das Blutströmungsrohrs (8) herumgewickelt ist.

6. Blutaufreinigungssäule nach einem der Ansprüche 1 bis 5, wobei der Adsorptionsmittelträger (5) aus einer Faser besteht, auf der Antibiotikum immobilisiert ist.

7. Blutaufreinigungssäule nach Anspruch 6, wobei das Antibiotikum Polymyxin ist.


**Revendications**

1. Colonne de purification de sang (1) qui comprend :

   un corps cylindrique (2) ;
   un premier collecteur (3) qui ferme une extrémité dudit corps cylindrique (2) et qui possède un premier canal de sang (9) qui communique avec l'intérieur dudit corps cylindrique (2) ;
   un second collecteur (4) qui ferme l'autre extrémité dudit corps cylindrique (2) et qui possède un second canal de sang (10) qui communique avec l'intérieur dudit corps cylindrique (2) ;

un support absorbant (5) contenu dans ledit corps cylindrique (2) ;

une première plaque d'extrémité (6) prévue au niveau de l'extrémité du premier collecteur dudit corps cylindrique (2) ;

une seconde plaque d'extrémité (7) prévue au niveau de l'extrémité du second collecteur dudit corps cylindrique (2) ; et

un tube d'écoulement de sang cylindrique (8) qui s'étend dans la partie centrale dudit corps cylindrique (2) entre ladite première plaque d'extrémité (6) et ladite seconde plaque d'extrémité (7), plusieurs ouvertures (11) étant formées dans la surface circonférentielle dudit tube d'écoulement de sang cylindrique (8) ;

dans laquelle :

une extrémité dudit tube d'écoulement de sang (8) communique avec ledit premier canal de sang (9) ;

l'autre extrémité dudit tube d'écoulement de sang (8) est fermée ;

ladite première plaque d'extrémité (6) est installée de sorte que sa surface circonférentielle extérieure vient en contact intime avec la surface circonférentielle intérieure dudit corps cylindrique (2) ;

un espace (14) est prévu entre la surface circonférentielle extérieure de ladite seconde plaque d'extrémité (7) et la surface circonférentielle intérieure dudit corps cylindrique (2) ;

le rapport entre le diamètre extérieur dudit tube d'écoulement de sang (8) dans la section transversale verticale par rapport à la direction longitudinale D1 et le diamètre intérieur dudit corps cylindrique (2) dans la section transversale verticale par rapport à la direction longitudinale D2 est de 0,35 à 0,50 ; et

la capacité de sang est de 6 à 10 ml.

2. Colonne de purification de sang selon la revendication 1, dans laquelle le temps de séjour est de 0,9 à 10 minutes lorsque le débit du sang entrant est de 3 à 10 ml/minute.

3. Colonne de purification de sang selon la revendication 1 ou 2, dans laquelle le rapport d'aire ouverte dudit tube d'écoulement de sang (8) est de 15 à 85%.

4. Colonne de purification de sang selon les revendications 1 à 3, dans laquelle ledit support absorbant (5) est un tissu tricoté.

5. Colonne de purification de sang selon la revendication 4, dans laquelle ledit tissu tricoté est enroulé autour dudit tube d'écoulement de sang (8).

6. Colonne de purification de sang selon l'une quelconque des revendications 1 à 5, dans laquelle ledit support absorbant (5) est composé d'une fibre enduite d'un antibiotique.

7. Colonne de purification de sang selon la revendication 6, dans laquelle ledit antibiotique est la polymyxine.

[Fig. 1]

[Fig. 2]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 1671925 B
- JP 2853452 B

- EP 1066843 A1